# EUROPEAN PATENT SPECIFICATION

(11) **EP 2 262 547 B1**
(45) Date of publication and mention of the grant of the patent: **09.01.2013**
(21) Application number: 09717854.5
(22) Date of filing: 06.03.2009
(51) Int. Cl.: A61L 29/04, A61L 29/08, A61L 29/16

(54) **BALLOON CATHETER DEVICES WITH SOLVENT-SWELLABLE POLYMER**
BALLONKATHETERVORRICHTUNGEN MIT IN LÖSUNGSMITTEL QUELLBAREM POLYMER
DISPOSITIFS DE CATHÉTER À BALLONNET AVEC UN POLYMÈRE GONFLABLE DANS UN SOLVANT

(30) Priority: 06.03.2008 US 34328
(43) Date of publication of application: 22.12.2010
(73) Proprietor: Boston Scientific Scimed, Inc., Maple Grove, MN 55311-1566 (US)
(72) Inventor: CHEN, John, Plymouth Minnesota 55446 (US)
(74) Representative: Vossius & Partner
(86) International application number: PCT/US2009/036349
(87) International publication number: WO 2009/111709

(56) References cited:
- EP-A1- 1 462 127
- US-A1- 2008 021 385

## Description

The present invention relates to medical devices, more particularly, to catheter devices.

Catheters are used in a wide variety of minimally-invasive or percutaneous medical procedures. Balloon catheters having drug coatings may be used to treat diseased portions of blood vessels. Typically, the balloon is inserted through a peripheral blood vessel and then guided via a catheter through the vascular system to the target intravascular site. However, as the balloon travels through the vascular system, the flow of blood may wash away some of the drug coating. In addition, the control of the timing, location and/or duration of the release of the drug can be an issue. Therefore, there is a need for improved catheter-based devices for drug delivery to an intravascular site. US 2008/021385 A1 discloses a balloon catheter coated with a crosslinked polymer, wherein a drug is applied by immersing into a drug solution. EP 1 462 127 A1 discloses a coated substrate, such as a dilation balloon, wherein a drug is associated with the coating by for example interpenetration by solvent swelling.

In one embodiment, the present invention provides a method for making a medical device, comprising: providing a balloon, wherein a wall of the balloon or a coating over the balloon comprises a polymer, wherein the polymer is swellable in an organic solvent, wherein the polymer is a semi-crystalline polymer comprising crystalline and amorphous regions, exposing said polymer to a mixture of said solvent and a therapeutic agent; and removing the solvent.

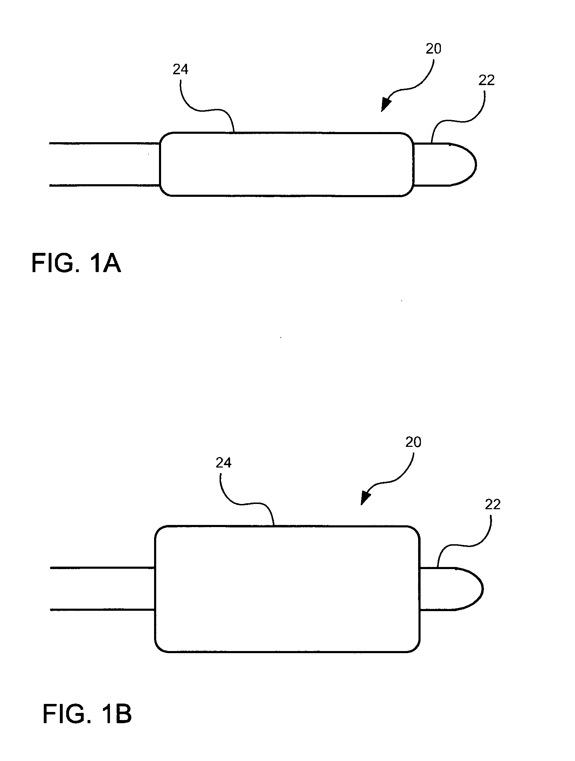

**FIGS. 1A** and **1B** show a catheter device according to an embodiment of the present invention. **FIG. 1A** shows the catheter device with the balloon in a deflated state. **FIG. 1B** shows the catheter device with the balloon in an inflated state.

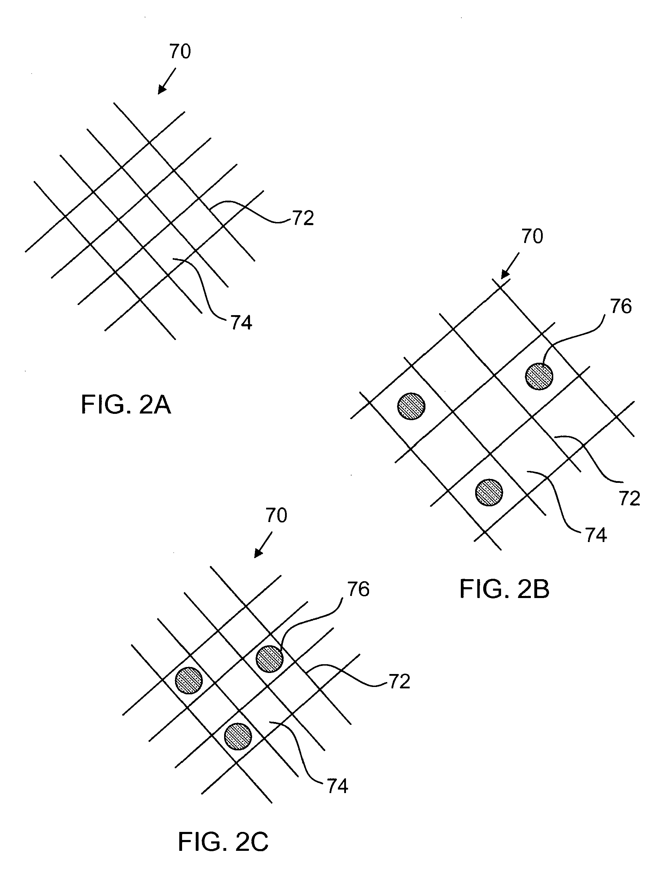

**FIGS. 2A-2C** schematically illustrate a polymer matrix in a balloon wall or a balloon coating according to an embodiment of the present invention. **FIG. 2A** shows the polymer matrix prior to solvent exposure. **FIG. 2B** shows the polymer matrix in an expanded condition after exposure to a mixture of a solvent and a therapeutic agent. **FIG. 2C** shows the polymer matrix in a contracted condition after the removal of the solvent.

Catheter devices of the present invention use an expandable balloon for delivering a therapeutic agent to a target site in the body. The balloon is designed to be insertable in the body via a catheter. The therapeutic agent can be associated with the balloon in any of various ways, as further described below. Any of various mechanisms conventionally used for the delivery, actuation, or expansion (e.g., by inflation) of balloon catheter devices may be used in the present invention. The balloon catheter may be designed similar to those that have been known in the art, including but not limited to angioplasty catheters, stent delivery catheters, inflation catheters and/or perfusion catheters. The catheter devices of the present invention may be used in conjunction with other drug delivery devices, such as stents.

Referring to **FIGS. 1A** and **1B**, in certain embodiments, a balloon catheter **20** comprises a catheter body **22** having a balloon **24** mounted thereon. In **FIG. 1A**, the balloon is in a deflated state; in **FIG. 1B**, the balloon is in an inflated state. The body of the balloon can be single-layered or multiple-layered.

In one aspect of the present invention, a balloon comprises a solvent-swellable polymer. The solvent-swellable polymer may have a therapeutic agent incorporated therein and may be used to form a wall of the balloon or to form a coating disposed over the balloon.

The polymer is swellable upon exposure to an organic solvent. The organic solvent can be polar or non-polar. Preferably, the organic solvent has a low boiling point. Preferably, the organic solvent has a boiling point of about 90°C or less, about 80°C or less, or about 70°C or less. Solvents with low boiling points can be removed relatively casier than those with high boiling points. Non-limiting examples of organic solvents include dichloromethane, chloroform, carbon tetrachloride, ethyl acetate, tetrahydrofuran, acetonitrile, hexane, and cyclohexane.

The solvent-swellable polymer is a semi-crystalline polymer. The semi-crystalline polymer can form a polymer matrix having crystalline portions and amorphous portions. When the polymer matrix is exposed to a mixture of a suitable organic solvent and a therapeutic agent, the solvent and the therapeutic agent diffuses into and swells preferentially the amorphous regions of the polymer matrix. The crystalline portions of the polymer matrix are more resistant to swelling, and therefore form a stable structure that maintains the physical structure of the polymer-containing balloon wall or coating. When the solvent is removed, the polymer matrix contracts to its original conformation, entrapping the therapeutic agent within the polymer matrix.

For example, referring to the schematic illustration shown in **FIGS. 2A-2C**, a catheter device comprises a balloon comprising a solvent-swellable, semi-crystalline polymer. **FIG. 2A** shows a polymer matrix **70** on the wall of the balloon prior to solvent exposure. Polymer matrix **70** has crystalline regions **72**, which are represented schematically by the lines, and amorphous regions **74**, which are represented schematically by the spaces. Referring to **FIG. 2B**, upon exposure to a mixture of a solvent and a therapeutic agent, amorphous regions **74** absorb the solvent and therapeutic agent **76**, causing polymer matrix **70** to swell. Referring to **FIG. 2C**, upon removal of the solvent, polymer matrix **70** contracts to its original conformation such that therapeutic agent **76** is retained within polymer matrix **70**.

Optionally, the solvent-swellable polymer is a partially cross-linked polymer. Preferably, the cross-linking density is low to allow at least a portion of the polymer to swell in an organic solvent. The cross-linking can be done using any known methods. For example, the cross-linking may be induced by UV irradiation or electron beam irradiation. The cross-linked portions of the polymer serve as the stable structure, while the non-crosslinked portions of the polymer swell upon exposure to an organic solvent and entrap the therapeutic agent previously mixed with the solvent after the solvent is removed.

Optionally, the solvent-swellable polymer is grafted onto the surface of the balloon through chemical bonding. In this case, the balloon wall base itself serves as the stable structure, while the polymer swells upon exposure to an organic solvent and entraps the therapeutic agent previously mixed with the solvent after the solvent is removed.

Non-limiting examples of solvent-swellable polymers include block copolymers of styrene-ethylene/butylene-styrene ("SEBS"), block copolymers of styrene-ethylene/propylene-styrene ("SEPS"), block copolymers of styrene-butadiene-styrene ("SBS"), block copolymers of styrene-isoprene-styrene ("SIS"), polyolefins such as polyethylene and polypropylene, polyurethane, polyoxymethylene-acetyl copolymers, polyamide block copolymers, and copolymers of acrylates and methacrylates. The SEBS, SEPS, SBS, and SIS copolymers can be commercially-available copolymers, such as those sold under the trade names KRATON G and KRATON D. Preferably, the solvent-swellable polymer has unsaturated bonds to allow for cross-linking or grafting.

After the therapeutic agent is loaded into the polymer, the solvent may be removed by any known method, for example, evaporation or vacuum drying. Preferably, the solvent is removed without heating. Preferably, the solvent is removed by evaporation.

In operation, the balloon comprising a therapeutic agent-containing polymer is inserted into the body and delivered to the target site. The balloon is then inflated, causing the polymer (in the case of a semi-crystalline polymer being used, polymer matrix **70**) in the balloon wall to undergo expansion by mechanical force. By enlarging the space within the polymer (e.g. polymer matrix **70**), the therapeutic agent (e.g. therapeutic agent **76**) is released from the wall of the balloon. Because the therapeutic agent is preferably loaded through diffusion, it tends to locate near the surface of the wall or coating and can be easily released after the balloon catheter device is delivered to the target site.

Medical devices of the present invention may also include a vascular stent mounted on the balloon. The vascular stent may be any of those known in the art, including those with or without coatings that contain a therapeutic agent. The stent may also be biostable, bioerodable, or biodegradable.

The balloons of the present invention may also be coated with a low-molecular weight carbohydrate, such as mannitol. The carbohydrate may be a separate coating or be blended with the therapeutic agent. The balloons of the present invention may also be coated with a radiocontrast agent (ionic or non-ionic), such as iopromide. The contrast agent may be a separate coating or be blended with the therapeutic agent.

The therapeutic agent used in the present invention may be any pharmaceutically acceptable agent (such as a drug), a biomolecule, a small molecule, or cells. Example drugs include anti-proliferative agents or anti-restenosis agents such as paclitaxel, sirolimus (rapamycin), tacrolimus, everolimus, and zotarolimus. Other example drugs include those that are anti-spasmodic agents and vasodilators. Example biomolecules include peptides, polypeptides and proteins; antibodies; oligonucleotides; nucleic acids such as double or single stranded DNA (including naked and cDNA), RNA, antisense nucleic acids such as antisense DNA and RNA, small interfering RNA (siRNA), and ribozymes; genes; carbohydrates; angiogenic factors including growth factors; cell cycle inhibitors; and anti-restenosis agents. Example small molecules include hormones, nucleotides, amino acids, sugars, and lipids and compounds have a molecular weight of less than 100kD. Example cells include stem cells, progenitor cells, endothelial cells, adult cardiomyocytes, and smooth muscle cells.

### Example 1

A double-layered balloon is manufactured. The inner layer is made of high density polyethylene and the outer layer is made of SEBS (KRATON G, Kraton Polymers, Houston, TX). The SEBS in the outer layer is crosslinked by electron beam irradiation. The balloon is then immersed in a mixture of cyclohexane and paclitaxel for drug loading. After the balloon is taken out, the cyclohexane is removed by evaporation. The balloon is then assembled onto a balloon catheter device.

## Claims

1. A method for making a medical device, comprising:
providing a balloon, wherein a wall of the balloon or a coating over the balloon comprises a polymer forming a polymer matrix, wherein the polymer matrix is swellable in an organic solvent, wherein the polymer is a semi-crystalline polymer comprising crystalline regions and amorphous regions.
exposing said polymer matrix on the balloon to a mixture of said solvent and a therapeutic agent, thereby swelling the polymer matrix and loading the therapeutic agent into the polymer matrix; and
removing the solvent.

2. The method of claim 1, wherein a wall of the balloon comprises the polymer swellable in an organic solvent.

3. The method of claim 1, wherein a coating over the balloon comprises the polymer swellable in an organic solvent.

4. The method of claim 3, wherein the polymer is grafted to a wall of the balloon through chemical bonding.

5. The method of claim 1, wherein the polymer is partially cross-linked.

6. The method of claim 1, wherein the polymer is selected from the group consisting of block copolymers of styrene-ethylene/butylene-styrene, block copolymers of styrene-ethylene/propylene-styrene, block copolymers of styrene-butadiene, block copolymers of styrene-isoprene, polyolefins, polyurethane, polyoxymethylene-acetyl copolymers, polyamide block copolymers, copolymers of acrylates and methacrylates, and mixtures thereof.

7. The method of claim 6, wherein the polymer is selected from the group consisting of block copolymers of styrene-ethylene/butylene-styrene, block copolymers of styrene-ethylene/propylene-styrene, block copolymers of styrene-butadiene, block copolymers of styrene-isoprene, polyurethane, and mixtures thereof.

8. The method of claim 1, wherein the solvent is a polar organic solvent.

9. The method of claim 1, wherein the solvent is a non-polar organic solvent.

10. The method of claim 1, wherein the boiling point of the solvent is about 90°C or less.

11. The method of claim 1, wherein the solvent is selected from the group consisting of dichloromethane, chloroform, carbon tetrachloride, ethyl acetate, tetrahydrofuran, acetonitrile, hexane, cyclohexane, and mixtures thereof.

12. The method of claim 1, wherein the solvent is removed by evaporation.

13. The method of claim 1, wherein the therapeutic agent is selected from the group consisting of paclitaxel, sirolimus, tacrolimus, everolimus, zotarolimus, and mixtures thereof.

## Patentansprüche

1. Ein Verfahren zur Herstellung einer medizinischen Vorrichtung, umfassend:
Bereitstellen eines Ballons, wobei eine Wand des Ballons oder eine Beschichtung auf dem Ballon ein Polymer umfasst, welches eine Polymermatrix bildet, wobei die Polymermatrix in einem organischen Lösungsmittel quellfähig ist, wobei das Polymer ein halbkristallines Polymer, umfassend kristalline Bereiche und amorphe Bereiche, ist;
Aussetzen der Polymermatrix auf dem Ballon einem Gemisch des Lösungsmittels und eines therapeutischen Mittels, dadurch Aufquellen der Polymermatrix und Beladen der Polymermatrix mit dem therapeutischen Mittel; und
Entfernen des Lösungsmittels.

2. Das Verfahren nach Anspruch 1, wobei eine Wand des Ballons das in einem organischen Lösungsmittel quellfähige Polymer umfasst.

3. Das Verfahren nach Anspruch 1, wobei eine Beschichtung auf dem Ballon das in einem organischen Lösungsmittel quellfähige Polymer umfasst.

4. Das Verfahren nach Anspruch 3, wobei das Polymer durch eine chemische Bindung auf die Wand des Ballons aufgepfropft ist.

5. Das Verfahren nach Anspruch 1, wobei das Polymer teilweise vernetzt ist.

6. Das Verfahren nach Anspruch 1, wobei das Polymer aus der Gruppe, bestehend aus Blockcopolymeren von Styrol-Ethylen/Butylen-Styrol, Blockcopolymeren von Styrol-Ethylen/Propylen-Styrol, Blockcopolymeren von Styrol-Butadien, Blockcopolymeren von Styrol-Isopren, Polyolefinen, Polyurethan, Polyoxymethylen-Acetyl-Copolymeren, Polyamid-Blockcopolymeren, Copolymeren von Acrylaten und Methacrylaten und Gemischen davon, ausgewählt ist.

7. Das Verfahren nach Anspruch 6, wobei das Polymer aus der Gruppe, bestehend aus Blockcopolymeren von Styrol-Ethylen/Butylen-Styrol, Blockcopolymeren von Styrol-Ethylen/Propylen-Styrol, Blockcopolymeren von Styrol-Butadien, Blockcopolymeren von Styrol-Isopren, Polyurethan und Gemischen davon, ausgewählt ist.

8. Das Verfahren nach Anspruch 1, wobei das Lösungsmittel ein polares organisches Lösungsmittel ist.

9. Das Verfahren nach Anspruch 1, wobei das Lösungsmittel ein unpolares organisches Lösungsmittel ist.

10. Das Verfahren nach Anspruch 1, wobei der Siedepunkt des Lösungsmittels etwa 90°C oder weniger beträgt.

11. Das Verfahren nach Anspruch 1, wobei das Lösungsmittel aus der Gruppe, bestehend aus Dichlormethan, Chloroform, Tetrachlorkohlenstoff, Ethylacetat, Tetrahydrofuran, Acetonitril, Hexan, Cyclohexan und Gemischen davon, ausgewählt ist.

12. Das Verfahren nach Anspruch 1, wobei das Lösungsmittel durch Verdampfen entfernt wird.

13. Das Verfahren nach Anspruch 1, wobei das therapeutische Mittel aus der Gruppe, bestehend aus Paclitaxel, Sirolimus, Tacrolimus, Everolimus, Zotarolimus und Gemischen davon, ausgewählt ist.

## Revendications

1. Procédé pour fabriquer un dispositif médical comprenant:
fournir un ballonnet, dans lequel une paroi du ballonnet ou un revêtement sur le ballonnet comprend un polymère formant une matrice polymère, dans lequel la matrice polymère est gonflable dans un solvant organique, dans lequel le polymère est un polymère semi-cristallin comprenant des régions cristallines et des régions amorphes ;
exposer ladite matrice polymère sur le ballonnet à un mélange dudit solvant et d'un agent thérapeutique, gonflant ainsi la matrice polymère et chargeant l'agent thérapeutique dans la matrice polymère ; et
retirer le solvant.

2. Procédé selon la revendication 1, dans lequel une paroi du ballonnet comprend le polymère gonflable dans un solvant organique.

3. Procédé selon la revendication 1, dans lequel un revêtement sur le ballonnet comprend le polymère gonflable dans un solvant organique.

4. Procédé selon la revendication 3, dans lequel le polymère est greffé sur une paroi du ballonnet par liaison chimique.

5. Procédé selon la revendication 1, dans lequel le polymère est partiellement réticulé.

6. Procédé selon la revendication 1, dans lequel le polymère est choisi dans le groupe constitué par les copolymères blocs de styrène-éthylène/butylène-styrène, les copolymères blocs de styrène-éthylène/propylène-styrène, les copolymères blocs de styrène-butadiène, les copolymères blocs de styrène-isoprène, les polyoléfines, le polyuréthane, les copolymères de polyoxyméthylène-acétyle, les copolymères blocs de polyamide, les copolymères d'acrylates et méthacrylates, et les mélanges de ceux-ci.

7. Procédé selon la revendication 6, dans lequel le polymère est choisi dans le groupe constitué par les copolymères blocs de styrène-éthylène/butylène-styrène, les copolymères blocs de styrène-éthylène/propylène-styrène, les copolymères blocs de styrène-butadiène, les copolymères blocs de styrène-isoprène, le polyuréthane, et les mélanges de ceux-ci.

8. Procédé selon la revendication 1, dans lequel le solvant est un solvant organique polaire.

9. Procédé selon la revendication 1, dans lequel le solvant est un solvant organique non polaire.

10. Procédé selon la revendication 1, dans lequel le point d'ébullition du solvant est d'environ 90 °C ou moins.

11. Procédé selon la revendication 1, dans lequel le solvant est choisi dans le groupe constitué par le dichlorométhane, le chloroforme, le tétrachlorure de carbone, l'acétate d'éthyle, le tétrahydrofuranne, l'acétonitrile, l'hexane, le cyclohexane, et les mélanges de ceux-ci.

12. Procédé selon la revendication 1, dans lequel le solvant est retiré par évaporation.

13. Procédé selon la revendication 1, dans lequel l'agent thérapeutique est choisi dans le groupe constitué par le paclitaxel, le sirolimus, le tacrolimus, l'évérolimus, le zotarolimus, et les mélanges de ceux-ci.
